# EUROPÄISCHE PATENTSCHRIFT

(11) **EP 1 018 337 B1**
(45) Veröffentlichungstag und Bekanntmachung des Hinweises auf die Patenterteilung: **05.02.2003**
(21) Anmeldenummer: 99123468.3
(22) Anmeldetag: 25.11.1999
(51) Int. Cl.: A61K 31/155, A61K 47/26, A61P 31/02

(54) **Chlorhexidin-Formulierungen und neue Chlorhexidinsalze**
Formulations containing chlorhexidine and novel chlorhexidine salts
Compositions pharmaceutiques de chlorhexidine et nouveaux sels de chlorhexidine

(30) Priorität: 11.12.1998 DE 19857151
(43) Veröffentlichungstag der Anmeldung: 12.07.2000
(73) Patentinhaber: Degussa AG, 40474 Düsseldorf (DE)
(72) Erfinder: Werle, Peter, Dr., 63571 Gelnhausen (DE); Merz, Friedhelm, 55283 Nierstein (DE); Joergensen, Judith N., Tunbridge Wells, Kent TN2 3DX (GB); Trageser, Martin, 63571 Gelnhausen (DE)

(56) Entgegenhaltungen:
- EP-A- 0 181 161
- EP-A- 0 200 607
- WO-A-98/46217
- FR-A- 2 752 731

## Beschreibung

Die Erfindung betrifft Chlorhexidinbase enthaltende pulverförmige Formulierungen, welche in wäßrige Lösungen von Chlorhexidinsalzen überführt werden können. Die Formulierungen, Lösungen und Salze lassen sich als Desinfektionsmittel sowie zur Herstellung solcher verwenden.

Chlorhexidin, chemisch als 1,1'-Hexamethylen-bis-[5-(4-chlorphenyl)-biguanid anzusprechen, ist eine stark basisch wirkende Substanz mit nur sehr geringer Wasserlöslichkeit. Durch Umsetzung der Chlorhexidinbase mit einer Vielzahl von Säuren leiten sich ebenfalls schwer wasserlösliche Salze ab. Chlorhexidinbase und vor allem ihr wasserlösliches Salz mit D(+)-Gluconsäure [CAS-Nr. 526-95-4] stellen bedeutende antibakterielle Substanzen dar, sowohl für den Einsatz im Human- als auch im Tierbereich. Hervorzuheben sind die niedrige Toxizität und allgemeine Verträglichkeit mit kationischen und anionischen Detergentien. Das Chlorhexidindigluconat wird als 20 %ige wäßrige Lösung angeboten und ist die derzeit einzige kommerziell verfügbare wasserlösliche Form der Base. Flüssige Formulierungen des Chlorhexidindigluconats (CHD-Gluconat) werden vielfältig modifiziert und als antibakterieller Zusatz in Kosmetika, zur Hautdesinfektion, Wundbehandlung in der Veterinärmedizin als Euterdesinfektionsmittel und auch zur oberflächendesinfektion eingesetzt.

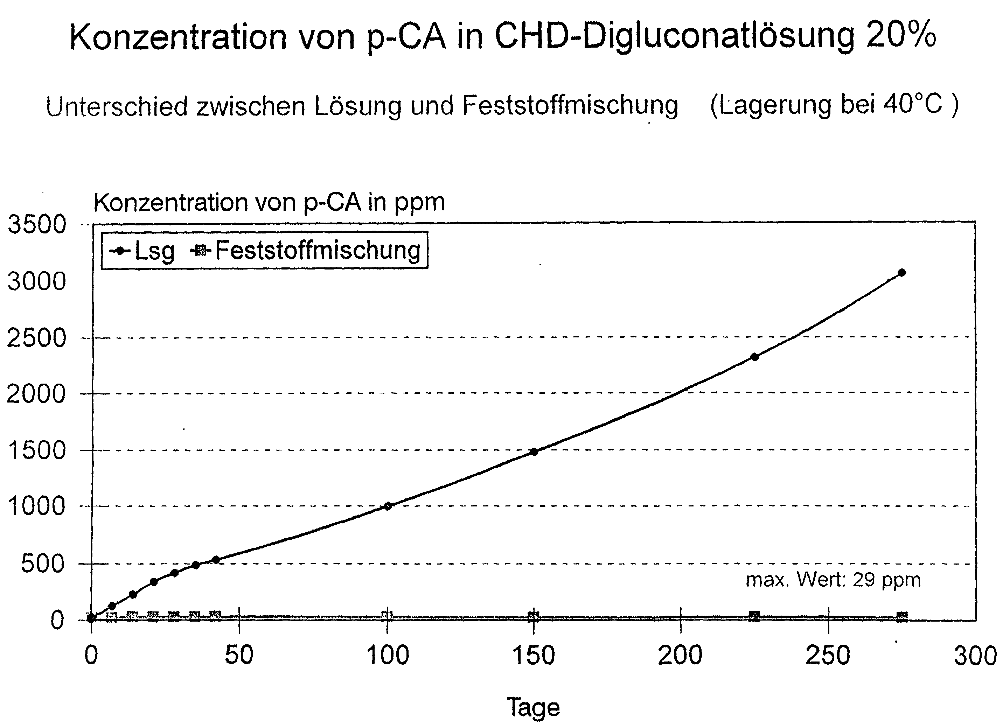

Die Gluconatlösungen unterliegen in ihrer Zusammensetzung und Erscheinung den Anforderungen des Europäischen Arzneibuches und der amerikanischen Pharmakopoe. Eine der Reinheitsforderungen ist der auf 500 ppm begrenzte Gehalt an p-Chloranilin. In Umkehrung der Bildung der Chlorhexidinbase aus Hexamethylenbicyanoguanidin und p-Chloranilin kann beim Lösen der Base in D(+) Gluconsäure-6-lacton, dem inneren Ester der D(+)-Gluconsäure, aus diesen Lösungen p-Chloranilin reversibel abgespalten werden, wobei sich die Lösungen zunehmend gelblich bis bräunlichen verfärben. Die Zersetzung von Chlorhexidingluconatlösungen ist abhängig vom pH der Lösung und vor allem der Lagertemperatur. Untersuchungen zeigen (Fig. 1), daß bei konstanter Lagerung bei 40 °C nach etwa einem Monat die zulässigen p-Chloranilinwerte überschritten werden. Langzeitstabile Lösungen von CHD-gluconat sind bislang nicht bekannt. Der Einsatz solcher Lösungen in Gebieten mit Tropenklima, ist daher problematisch und bislang nicht befriedigend gelöst. Es besteht daher ein Bedarf an Chlorhexidinsalzlösungen, die bei Verwendung unter ungünstigen klimatischen Bedingungen, insbesondere bei hohen Temperaturen, nur in geringem Maße zur Zersetzung neigen. Leider sind nahezu alle Salze des Chlorhexidins in Wasser schwer löslich oder können aufgrund der toxikologischen Eigenschaften des Anions nicht als Humanoder Veterinärdesinfektionsmittel eingesetzt werden. Schwer wasserlöslich sind beispielsweise Salze des Chlorhexidins mit Chlorwasserstoffsäure, Fluorphosphorsäure, Bishydroxymethylpropionsäure, Acetylsalicylsäure, Weinsäure, 4-Hydroxybenzoesäure, -5 Sulfosalicylsäure, Glyoxalsäure, Thioctsäure, L-Äpfelsäure, Sulfanilsäure, Nicotinsäure, Sarcosin, L(+)-Glutaminsäure, Citronensäure, Nitrilotriessigsäure, Trimethylolessigsäure, Sorbinsäure und viele mehr.

Mit Amidoschwefelsäure, Captopril, Lävulinsäure, N-Acetylglycin und S-(-)-Pyrolidinon-5-carbonsäure können zwar 20 %ige wässrige Lösungen erhalten werden, die aber beim Animpfen oder nach Stehen über einen längeren Zeitraum spontan auskristallisieren können. Chlorhexidinascorbat ist zwar gut wasserlöslich aber lichtempfindlich und instabiler als das Gluconat.

Aufgabe der Erfindung ist demgemäß, lagerstabile Formulierungen aufzuzeigen, die Chlorhexidin in wasserlöslicher Form enthalten. Die Formulierung sollte leicht herstellbar und in wäßrige Chlorhexidinsalzlösungen überführt werden können. Die zur Salzbildung benötigten Säuren sollen zudem toxikologisch unbedenklich sein.

Es wurde nun gefunden, daß sich gut wasserlösliche Salze von Chlorhexidin durch Umsetzung von Chlorhexidinbase mit den nachfolgenden Säuren oder Säurelactonen hiervon herstellen lassen:

Lactobionsäure (I) [CAS-Nr. 96-82-2], D-Galacton-γ-lacton (II) [CAS-Nr. 2782-07-2], L-Mannonsäure-γ-lacton (III) [CAS-Nr. 22430-23-5], D-(-)-Gulonsäure-γ-lacton (IV) [CAS-Nr. 6322-07-2], D-(+)-Galacturonsäure (V) [CAS-Nr. 91510-62-2] und α-D-Heptagluconsäure-γ-lacton (VI) [CAS-Nr. 60046-25-5].

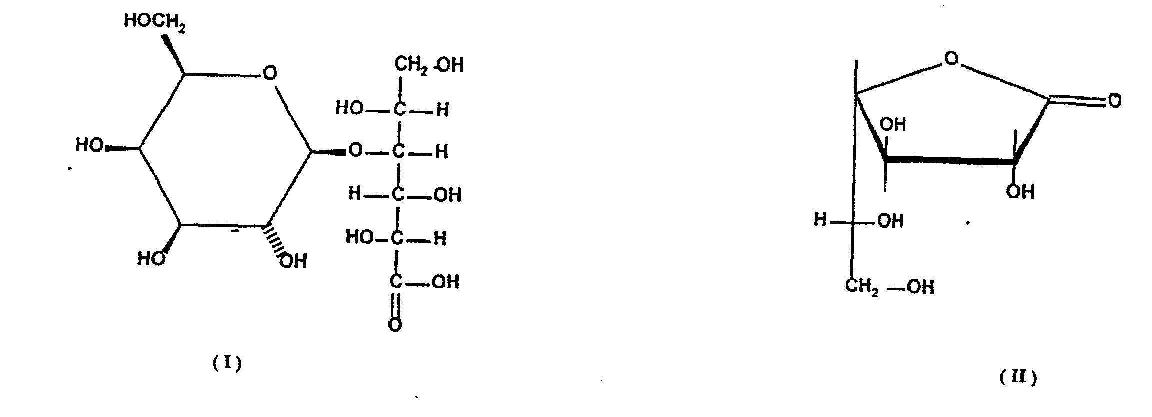

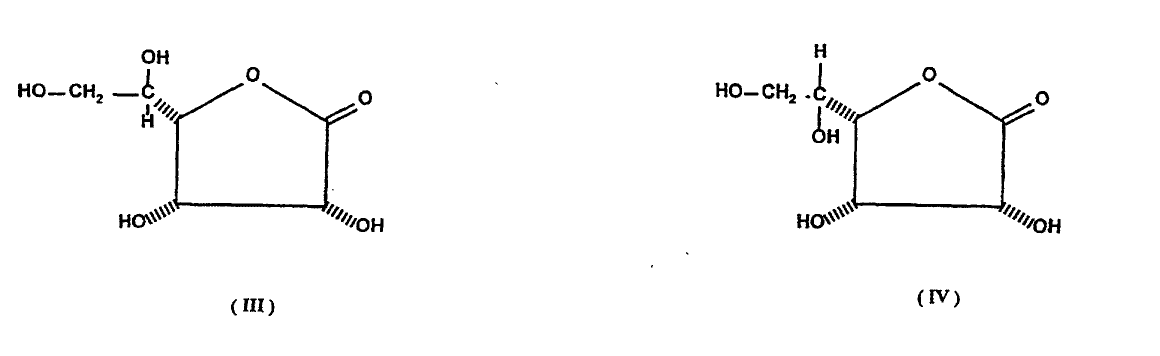

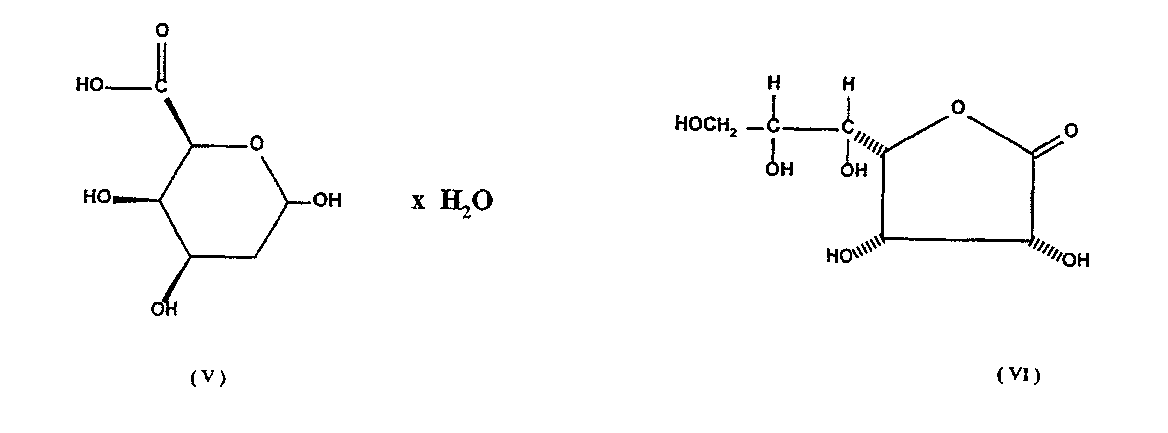

Gefunden wurde ferner, daß pulverförmige Gemische der Chlorhexidinbase mit ausgewählten Zuckersäuren oder Lactonen hiervon gemäß den Formeln (I) bis (VI) sowie Gluconsäure oder Gluconolacton wesentlich lagerstabiler sind als die Lösungen der aus den Gemischen durch Auflösen in Wasser leicht zugänglichen wäßrigen Lösungen der Chlorhexidinsalze. Gegenstand der Erfindung ist somit die Chlorhexidinformulierung gemäß Anspruch 1.

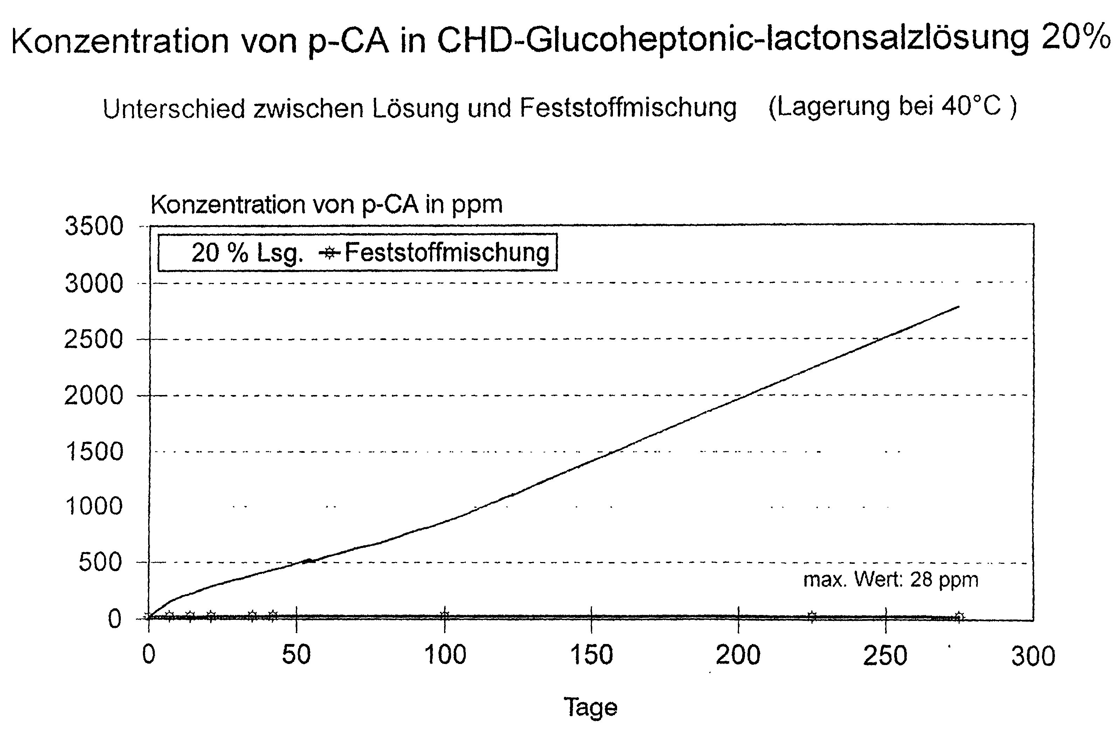

Lagerversuche an Salzlösungen haben gezeigt, daß ihre Lagerstabilität derjenigen von Chlorhexidingluconatlösungen vergleichbar sind. Das Problem der langfristigen p-Chloranilinabspaltung läßt sich durch die Einführung der neuen Anionen nicht lösen. Überraschenderweise sind jedoch erfindungsgemäße pulverförmige Formulierungen sehr lagerstabil - siehe Fig. 1 und 2.

Fig. 1 zeigt die Bildung von p-Chloranilin (pCA) in wässrigen 20 gew.-%igen Chlorhexidindigluconatlösungen sowie des entsprechenden pulverförmigen Gemischs in Zeitabhängigkeit.

Fig. 2 zeigt die Bildung von p-Chloranilin aus einer Lösung von Chlorhexidin-di-D-(-)-heptagluconat und des erfindungsgemäßen pulverförmigen Gemischs enthaltend Chlorhexidin und Glucoheptonic-lacton.

Es wurde nun gefunden, daß sich p-chloranilinarme Chlorhexidindigluconatlösungen oder Lösungen mit den Zuckersäurenanionen auf der Basis von (I) bis (VI) dadurch herstellen lassen, daß man feinpulvrige Mischungen von Chlorhexidinbase mit Gluconsäure oder Gluconolacton sowie mit einer Zuckersäure beziehungsweise deren Lacton (I) bis (VI) im Verhältnis Base : Säure/Lacton von 1 zu 2 bis 1 zu größer 2, insbesondere 1 zu 2,05 bis 2,6 durch Zugabe der benötigten Menge Wasser zu 20 %igen Lösungen des jeweiligen Salzes umsetzen kann, wobei unter Einsatz von Formulierungen mit den Zuckersäuren (I), (V) oder (VI') die Lösung unter gelegentlichem Schütteln innerhalb von etwa 20 min. bei Raumtemperatur und unter Einsatz von Formulierungen mit den Zuckerlactonen (II), (III) oder (IV) durch Verwendung von warmem Wasser eintritt.

Die erfindungsgemäßen pulverförmigen Formulierungen sind über einen langen Zeitraum, auch bei höheren Temperaturen lagerfähig ohne p-Chloranilin abzuspalten - siehe Figur 1 und 2. Um Verklumpung der Formulierung zu vermeiden, sollte der Gehalt an freiem Wasser niedrig sein, vorzugsweise unter 0,05 %.

Die Mischungen aus Chlorhexidinbase und einer Zuckersäure oder Zuckersäurelacton werden durch sorgfältige Homogenisierung in geeigneten Apparaturen wie Taumelmischer, Draismischer, gewonnen und anschließend vermahlen. Alternativ ist es auch möglich, beide Komponenten im berechneten Verhältnis dem Mahlorgan getrennt zuzuführen.

Hier eignet sich beispielhaft eine Spiralstrahlmühle. Auch können Base und Säure/Lacton getrennt gemahlen und anschließend in der Stöchiometrie richtig eingestellt in ein Behältnis gegeben werden. Letztere Modifikation eignet sich vorzugsweise bei der Abfüllung in sog. Portionspackungen, die auf einmal verbraucht werden und so in sich nicht homogen zu sein brauchen, da beim Auflösen in Wasser sich automatisch das richtige Verhältnis Säure/Lacton: Base einstellt. Bei Bedarf kann die Formulierung auch 0-10 Gew.-%, vorzugsweise weniger als 1 Gew.-%, Hilfsstoffe, wie Duftstoffe, Farbstoffe, andere Desinfektionsmittel und Tenside zugesetzt werden.

Für eine rasche Auflösung ist es zweckmäßig, die Korngröße der eingesetzten Komponenten möglichst feinteilig einzusetzen. Vorzugsweise empfiehlt sich eine Korngröße von d₅₀ < 50 µm.

Es ist auch möglich, aus den aus Chlorhexidinbase und Zuckersäure oder Zuckerlacton hergestellten wässrigen Lösungen die Chlorhexidinsalze in kristalliner Form zu gewinnen. Zur Herstellung des festen Salzes wird die Lösung eingedampft, vorzugsweise unter vermindertem Druck; nach längerem Stehen der hochviskosen Masse über einem Trockenmittel versprödet die Masse und kann zu Pulver zerrieben werden. Gemäß einer alternativen Ausführungform wird eine konzentrierte wässrige Lösung des Chlorhexidinsalzes einer Tieftemperaturvakuumsublimation (Gefriertrocknung bei zum Beispiel -60 °C) unterworfen. Durch solch einen Prozess werden unmittelbar die kristallinen Salze, welche spontan wasserlöslich sind, erhalten.

Die erfindungsgemäßen Formulierungen und Chlorhexidinsalze lassen sich als Desinfektionsmittel oder zur Herstellung solcher verwenden.

### Beispiele

1. Die Herstellung der Salzlösungen erfolgt durch Zusammengeben der Komponenten (I) bis (VI) mit der berechneten Menge Wasser und Chlorhexidinbase.
a) Vorschrift zur Herstellung von wässrigen, 20 gew.-%igen Lösungen von Chlorhexidinsalzen durch Umsetzen der Zuckersäuren von (I), (V) und (VI') mit Chlorhexidinbase. 35,8 g (I) bzw. 21,2 g (V) bzw. 21 g (VI') werden zusammen mit 25,0 g Chlorhexidinbase zu 243 g bzw. 185 g bzw. 180 g Wasser gegeben und die sich bildende milchige Suspension etwa 10-15 min. bei Raumtemperatur gerührt, bei Bedarf ist der pH durch weiteren Zusatz von (I), (V) oder (VI') auf 5-6 einzustellen. Die resultierende klare Lösung enthält jeweils 20 Gew.-% des entsprechenden Chlorhexidinsalzes.
b) Vorschrift zur Herstellung von wässrigen 20 gew.-%igen Lösungen von Chlorhexidinsalzen durch Umsetzen der Zuckersäurelactone von (II), (III), (IV) mit Chlorhexidinbase.
19,5 g (I) bzw. (III), (IV) werden mit 25,0 g Chlorhexidinbase zu 171 g Wasser gegeben und die resultierende Suspension auf 60-80 °C für etwa 5-10 min. erwärmt. Bei Bedarf ist der pH durch weiteren Zusatz von (II), (III), (IV) auf 5-6 einzustellen. Man erhält klare 20 gew.-%ige Lösungen der entsprechenden Chlorhexidinsalze.
2. Herstellung von Chlorhexidinsalzen
a) 10,0 g Chlorhexidinbase und 8,3 g D-Heptagluconsäure-γ-lacton wurde in 35 ml Wasser gelöst. Die Lösung wurde im Vakuum zur Trockene eingedampft. Nach Stehenlassen über P₄O₁₀ erhielt man das Salz Chlorhexidin-heptagluconat als farblose kristalline Masse mit einem Schmelzbereich von 72 bis 76 °C.
b) In analoger Weise wurden weitere Salze Chlorhexidin-heptagluconat als Chlorhexidin(CH)-salze erhalten, deren Schmelzbereiche sind:

| | |
|---|---|
| CH-lactobionat | 100 - 115 °C |
| CH-galacturonat | 118 - 125 °C |
| CH-galactonat | 140 - 145 °C. |

## Patentansprüche

1. Chlorhexidinformulierung in Form eines pulverförmigen wasserlöslichen Gemischs, bestehend aus Chlorhexidinbase, einer oder mehreren Zuckersäuren oder Lactonen hiervon aus der Reihe Gluconsäure oder Gluconolacton, Lactobionsäure (I), D-Galacton-γ-lacton (II), L-Mannonsäure-γ-lacton (III), D-(-)-Gulonsäure-γ-lacton (IV), D-(+)-Galacturonsäure (V) und α-D-Heptagluconsäure-γ-lacton (VI), wobei das Molverhältnis Chlorhexidinbase zu Zuckersäure oder einem Lacton hiervon 1 zu gleich oder größer 2 ist, und 0 bis 10 Gew.-% Hilfsmitteln.

2. Formulierung nach Anspruch 1,
**dadurch gekennzeichnet,**
**daß** das Molverhältnis Chlorhexidinbase zu Zuckersäure oder Lacton hiervon 1 zu 2,05 bis 2,6 ist und der Gehalt an Hilfsmitteln unter 1 Gew.-% beträgt.

## Claims

1. A chlorhexidine formulation in the form of a powdered, water-soluble mixture consisting of chlorhexidine base, one or more sugar acids or lactones thereof from the set gluconic acid or gluconolactone, lactobionic acid (I), D-galactono-γ-lactone (II), L-mannono-γ-lactone (III), D-(-)-gulono-γ-lactone (IV), D-(+)-galacturonic acid (V) and α-D-heptaglucono-γ-lactone (VI), wherein the molar ratio of chlorhexidine base to sugar acid or lactone thereof is 1 to greater than or equal to 2, and 0 to 10 wt.% of auxiliary substances.

2. A formulation according to Claim 1,
**characterised in that**
the molar ratio of chlorhexidine base to sugar acid or lactone thereof is 1 : 2.05 to 2.6 and the concentration of auxiliary substances is less than 1 wt.%.

## Revendications

1. Formulation de chlorhexidine sous forme d'un mélange pulvérulent soluble dans l'eau, constitue de chlorhexidine base, d'un ou plusieurs acides de sucre ou des lactones de ceux-ci, de la série de l'acide gluconique ou de la gluconolactone, l'acide lactobionique (I), la D-galacto γ-lactone (II), la γ-lactone d'acide L-mannonique (III), la γ-lactone d'acide D(-)-gulonique (IV), l'acide D-(+)-galacturonlque (V) et la γ-lactone de l'acide α-heptagluconique (VI), pour lesquels le rapport molaire chlorhexidine base à l'acide de sucre ou à une lactone de celui-ci est 1 pour égal ou plus grand que 2, ainsi que de 0 à 10 % en poids d'adjuvants.

2. Formulation selon la revendication 1
**caractérisée en ce que**
le rapport molaire chlorhexidine base à l'acide de sucre ou à la lactone de celui-ci, est 1 pour 2,05 à 2,6 et la teneur en adjuvants s'élève à moins de 1 % en poids.
